(19) **Europäisches Patentamt / European Patent Office / Office européen des brevets**

(11) **EP 1 576 084 B1**

(12) **EUROPEAN PATENT SPECIFICATION**

(45) Date of publication and mention
of the grant of the patent:
**30.04.2008 Bulletin 2008/18**

(51) Int Cl.:
*C11D 17/00* (2006.01)    *B01J 2/00* (2006.01)
*C11D 3/386* (2006.01)

(21) Application number: **03814401.0**

(22) Date of filing: **24.12.2003**

(86) International application number:
**PCT/US2003/041638**

(87) International publication number:
**WO 2004/058933 (15.07.2004 Gazette 2004/29)**

(54) **MECHANICALLY ROBUST PLASTICIZED GRANULES**

WEICHGEMACHTE, MECHANISCH STABILE ROBUSTE GRANULATE

GRANULES PLASTIFIES ROBUSTES EN TERMES MECANIQUES

(84) Designated Contracting States:
**AT BE BG CH CY CZ DE DK EE ES FI FR GB GR HU IE IT LI LU MC NL PT RO SE SI SK TR**

(30) Priority: **24.12.2002 US 436206 P**
**28.05.2003 US 474086 P**

(43) Date of publication of application:
**21.09.2005 Bulletin 2005/38**

(73) Proprietor: **GENENCOR INTERNATIONAL, INC.**
**Palo Alto, California 94304 (US)**

(72) Inventors:
• **BECKER, Nathaniel, T.**
**Hillsborough, CA 94010 (US)**
• **GREEN, Thomas, S.**
**Palo Alto , California 94304 (US)**

• **GEBERT, Mark, S.**
**Pacifica, CA 94044 (US)**
• **MAZEAUD, Isabelle**
**Palo Alto ,California 94304 (US)**

(74) Representative: **Wibbelmann, Jobst et al**
**Wuesthoff & Wuesthoff**
**Patent- und Rechtsanwälte**
**Schweigerstrasse 2**
**81541 München (DE)**

(56) References cited:
| | |
|---|---|
| GB-A- 1 483 591 | US-A- 3 781 228 |
| US-A- 5 324 649 | US-A- 5 879 920 |
| US-A1- 2002 119 201 | US-A1- 2002 160 927 |
| US-A1- 2002 173 441 | US-B1- 6 432 902 |

Printed by Jouve, 75001 PARIS (FR)

**Description**

FIELD OF THE INVENTION

This invention relates to mechanically robust plasticized granules comprising an active ingredient, preferably an enzyme, and plasticizer material impregnating plasticizable components of the granule, such as a polymer coating, including processes for producing the granules with the impregnated plasticizer.

BACKGROUND OF THE INVENTION

Various industries, such as detergent manufacturing, pharmaceutical manufacturing, agrochemical manufacturing, and personal care manufacturing include compositions comprising active ingredients, particularly enzymes, that tend to form dust due to physical forces encountered during handling and blending operations. One of the problems with dust formation is that dust can cause health problems and allergic reactions. In an effort to protect the active ingredient and reduce dust formation, active ingredients have been formulated with various compounds including binders, coating agents, and various encapsulating agents. Numerous techniques have been developed to produce these formulations including prilling, extrusion, spheronization, drum granulation, and fluid bed spray coating. (See e.g. US-A-4,106,991; US-A-4,242,219; US-A-4,689,297; and US-A-5,324,649).

However, prior art formulations which produce particles or granules including an active ingredient do not always exhibit sufficient resistance to mechanical forces typically encountered during handling, such as impacts, shears and compressions, and as a result form dust when subjected to such physical forces.

US-A-5 879 920 describes granular enzyme compositions having reduced tendency to form dust and leave a residue, and improved stability and delayed release characteristics. The granules comprise a core, an enzyme layer and an outer coating layer comprising polymers selected from polyvinylpyrrolidone, polyvinylalcohol, polyethylene glycol. US 2002/0119201 A1 discloses active containing granules comprising a lubricant on the outer surface of the granule, the lubricant is a mineral oil or a nonionic surfactant.

Prior art formulations designed to improve the resistance of granules to impact and shear forces may include polymers as binders or coating agents. Plasticizers also may be added to improve the impact resistance of such granules; however, the use of plasticizers in granules and granule coatings is limited by their tendency to increase tackiness and agglomeration of formulations which incorporate polymers as coatings or binders. If used at all, plasticizers are typically limited to a range of less than 5% or 10% of the mass of the coating polymer and the plasticizer is added concurrently with the polymer coating, in the form of a coating solution or suspension, or as a molten mixture.

A continuing need exists in the art for mechanically robust granules that may be produced using procedures that do not result in undue tackiness and/or agglomeration, and thereby result in individual well-formed particles which are granular and free-flowing, and which can be coated or otherwise produced in an economical manner.

SUMMARY OF THE INVENTION

One aspect of the invention is a mechanically robust granule obtainable by the method of claim 1 comprising a mechanically sensitive particle including an active ingredient, a polymer film surrounding the mechanically sensitive particle, and a plasticizer applied to the particles after, and not concurrently with, formation of the granule. The plasticizer is applied at a temperature sufficient to allow the plasticizer to readily diffuse into the plasticizable components of the particle. The plasticizer may be poured or sprayed over or into a bed of granules with subsequent or concurrent agitation or mixing to spread and uniformly coat and soak into the granules. For example, the plasticizer may be spray coated onto a fluidized bed of particles. In one embodiment, the plasticizer is a mixture of components. In another embodiment the plasticizer is applied as an aqueous solution.

The plasticizable component is a water soluble or dispersible polymer or polymeric coating, for example, the plasticizable component is polyvinyl alcohol (PVA), gelatin, modified starch, such as hydroxypropylated cornstarch, cellulose ethers and derivatives and copolymers thereof, and particularly PVA and derivatives thereof. The plasticizer is a compound with a molecular weight less than 1000 Daltons, including glycerol, propylene glycol, polyethylene glycol, triethylene glycol, a sugar, or a sugar alcohol. In the case of granules with polymer coatings, plasticizers are selected based on their thermodynamic phase compatibility and ability to lower the glass transition temperature (Tg) of the specific polymer. Preferably, the active ingredient is a protein or peptide, preferably an enzyme selected from the group consisting of proteases, cellulases, amylases, lipases, cutinases and combinations thereof. The active ingredient may be incorporated into the core of the granule or preferably the active ingredient is layered over the core. The invention relates to a method according to claim 1.

The granule prepared using the above method has a reduced dust value measured by any test wherein mechanical forces are applied to the granules. For instance, the granule prepared using the above method has a reduced Repeated Impact Test (RIT) enzyme dust value of less than about 25,000 ng/g (test conditions of 60 Hz; 8.52 meters/sec velocity; and 216,000 collisions); a reduced Repeated Impact Test (RIT) enzyme dust value of less than about 400,000 ng/g (test conditions of 30 Hz; 3.2 meters/sec velocity; and 108,000 collisions); a reduced a Heubach total and enzyme dust value; and an reduced Elutriation dust value. As measured by any dust test, the granule prepared using the above method has at least a 25% enzyme dust reduction, at least a 35% enzyme dust reduction, at least a 45% enzyme dust reduction, at least a 55% enzyme dust reduction, at least a 65% enzyme dust reduction, at least a 75% enzyme dust reduction, at least a 85% enzyme dust reduction, at least a 95% enzyme dust reduction, and at least a 99% enzyme dust reduction, in comparison to the original unplasticized granule

In embodiments of the invention, the plasticizer is at least 100% absorbed, at least 95% absorbed, at least 90% absorbed, at least 85% absorbed, at least 80% absorbed, at least 75% absorbed, at least 70% absorbed, at least 65% absorbed, at least 60% absorbed, at least 55% absorbed, and at least 50% absorbed into the granule, where percent absorption is defined as the amount of plasticizer which permeates into the granule relative to the total amount of plasticizer applied to the granule, which can be determined by measuring the amount of unabsorbed plasticizer remaining on or outside the surface of the granule after application.

In a preferred embodiment of the method, the active ingredient is an enzyme, particularly an enzyme selected from the group of proteases, cellulases, amylases, cutinases, lipases and combinations thereof; the polymer is PVA and optionally glycerol is included as a plasticizer. In another aspect of the invention, a gelling agent is added as a component of the flexible film.

Another aspect of the invention relates to the use of the highly mechanically robust granules according to the invention to deliver active ingredients to an aqueous environment such as detergent active ingredients in a wash water.

In a further aspect the invention relates to compositions comprising the highly mechanically robust granules according to the invention.

DETAILED DESCRIPTION OF THE INVENTION

The present inventors have found that a granule obtainable by the method of claim 1 to which is applied a plasticizer having specific properties and applied in a specific manner to a previously coated particle comprising a plasticizable component, which include an active ingredient incorporated therein or which is surrounded by a layer including an active ingredient, can impart impact resistance to the particle. This results in a granule with reduced potential for dust formation because it is more resistant to mechanical forces during handling. The granules of the present invention are mechanically robust granules, which are made to deliver an active ingredient incorporated therein, particularly to an aqueous environment. The granules of the invention are very useful, for example in cleaning products, particularly detergent products, personal care products, fabric care products, and pharmaceutical products.

Unless defined otherwise, all technical and scientific terms used herein have the same meaning as commonly understood by one of ordinary skill in the art to which this invention pertains. As used in the specification and claims, the singular "a", "an" and "the" include the plural references unless the context clearly dictates otherwise. For example, the term granule may include a plurality of granules.

A "mechanically robust granule" according to the invention is defined as a granule with an impregnated plasticizer applied to a formed particle having a plasticizable component or coating, wherein the granule exhibits a significantly reduced dust value when subjected to repeated impacts, compressions or shear forces. For instance, a mechanically robust granule exhibits less than 50,000 ng/g of enzyme dust as measured by a Repeated Impact Test Device (RIT) with test conditions of 216,000 collisions at 8.52meters/second, 60Hz, a plastic material container, and an amplitude of 1.5 cm. (See USP 6,035,716). A mechanically robust granule according to the invention exhibits less than 400,000 ng/g of enzyme dust as measured by a RIT device with test conditions of 108,000 collisions at a velocity of 3.3 meters/second at 30 Hz using a metal container. A mechanically robust granule according to the invention exhibits less than 0.07 mg/gram total dust and less than 3.0 ug/gram of enzyme dust as measured by the Heubach dust test. A mechanically robust granule according to the invention exhibits less than 0.5 mg/gram of enzyme dust as measured by the Elutriation dust test. A mechanically robust granule according to the invention is also defined as a granule which has at least a 25% enzyme dust reduction, at least a 35% enzyme dust reduction, at least a 45% enzyme dust reduction, at least a 55% enzyme dust reduction, at least a 65% enzyme dust reduction, at least a 75% enzyme dust reduction, at least a 85% enzyme dust reduction, at least a 95% enzyme dust reduction, and at least a 99% enzyme dust reduction as compared to the same granule without an impregnated plasticizer coating. The term attrition as used herein includes breakdown of a granule within a process, and includes abrasion and fragmentation.

A "mechanically sensitive granule" is defined as a granule without an impregnated plasticizer, wherein the granule exhibits a dust value which indicates sensitivity to impact collisions and shear forces, namely, a granule with an RIT enzyme dust result of greater than 400,000 ng/gram (test conditions of 30 Hz; 3.3 meters/second; 108,000 collisions; using a metal container). For instance, a mechanically sensitive granule exhibits greater than 50,000 ng/g of dust as measured by a Repeated Impact Test Device (RIT) with test conditions of 216,000 collisions at 8.52m/s , 60 Hz, a plastic material container, and an amplitude of 1.5 cm (See USP 6,035,716). For instance, a mechanically sensitive granule exhibits greater than 0.1 mg/gram total dust and 5.0 ug/gram enzyme dust as measured by the Heubach dust test. For instance, a mechanically sensitive granule exhibits greater than 2.0 mg enzyme dust/pad as measured by the Elutriation dust test.

"Impregnated", in reference to plasticizers and for purposes of this disclosure, means that at least a substantial portion of the plasticizer has penetrated or soaked into, as opposed to laying on top of, at least the outer layer, or plasticizable component of the granule. Impregnating encompasses embedding, absorbing, diffusing into, and permeating at least the plasticizable component to securely incorporate the plasticizer within the granule. Furthermore, impregnation inherently involves addition of the plasticizer subsequent to, and not concurrent with, the formation of the granule and any polymer or coating layer on the granule. In embodiments of the invention, the plasticizer is at least 100% absorbed, at least 95% absorbed, at least 90% absorbed, at least 85% absorbed, at least 80% absorbed, at least 75% absorbed, at least 70% absorbed, at least 65% absorbed, at least 60% absorbed, at least 55% absorbed, and at least 50% absorbed into the granule.

"Glass Transition Temperature (Tg)" means the temperature at which an amorphous material (or the amorphous regions in a partially crystalline material) changes from a hard and relatively brittle condition to a viscous or rubbery condition. Plasticizers are known to lower the glass transition temperature of polymers or other materials, and specific plasticizers are known in the art to be effective in plasticizing particular polymers or materials.

"Plasticizable component" means for purposes of this disclosure an amorphous material capable of having a reduced Tg by the addition of a low molecular weight component, or plasticizer.

GRANULES

The granules according to the invention comprise an active ingredient and further an impregnated plasticizer diffused into a plasticizable component or layer of the granule. The active ingredient may be incorporated into a core or may be layered around the core preferably followed by a layer of a polymer film.

The granules of the invention are mechanically robust and exhibit low dust, as defined herein. The granules are stable when stored under ambient humidity and temperature conditions, but soluble or dispersible upon contact with water so as to release the active ingredient or part thereof upon contact with water.

Several dust tests have been developed to measure the mechanical resistance to attrition and dusting formation of different granular enzyme formulations. These include the Heubach attrition test and the elutriation test. The Heubach test subjects particles to defined crushing and fluidization forces by using rotating paddles to roll steel balls through a bed of granules contained within a cylindrical chamber and simultaneously percolating a stream of air through the bed to strip off any dust that is generated. The generated dust is drawn by vacuum through a tube and deposited onto a filter pad outside the Heubach chamber. The weight or active component of the dust collected is referred to as Heubach dust. In the elutriation test, granules are placed on a glass frit within a tall glass tube and fluidized with a constant dry airstream over a fixed period of time. A discussion of the principles, operation and limitations of the Heubach and elutriation dust tests can be found for example, in "Enzymes In Detergency" ed. Jan H. van Ee., Ch. 15, pgs. 310 - 312, (Marcel Dekker, Inc. New York (1997) and references cited therein.

While the Heubach and elutriation tests are in common usage, neither of these tests adequately models the isolated effect of impact forces upon granule integrity and attrition. For purposes of modeling attrition of particles caused by impact forces, particularly the effects of large numbers of repeated impacts of defined magnitude, the Repeated Impact Test (RIT) was developed. In this test a sample of granules is vibrated at a controlled frequency and amplitude within a chamber. The amount of damaged particles or fragments (RIT mass attrition) is measured, or after removing all the granules and broken granule fragments the dust generated (RIT dust) is extracted from the box with a buffer and assayed for enzyme activity (See WO 98/03849 and USP 6,035,716 which are incorporated by reference herein). Two different RIT protocols were used to conduct the experiments. In the high velocity protocol, the 30 mg granule samples were tested at 60 Hz, 8.52 meters/second velocity, and 218,000 collisions into a rigid, plastic material container wall. In the low velocity protocol, the 30 mg granule samples were tested at 30 Hz, 3.2 meters/second velocity, and 108 collisions into a rigid, metal material container wall.

Impregnated mechanically robust granules of the invention tend to be resistant to the high and low velocity impact forces and often as well to compression and shear forces typically encountered in various manufacturing operations, although the specific mode of failure under the slow strain rate of compression can be quite different than that seen under the high strain rate of typical impact forces. By virtue of the inclusion of impregnated plasticizer according to the invention, the resulting granules are well suited to readily absorb substantial and repeated impacts, shears, or compressions. The plasticizer-impregnated granule, preferably encompassing a plasticized polymer coating, tends to deform while maintaining its integrity, increasing the magnitude or number of attritional forces it can absorb before reaching a point of sudden failure, or reduce the amount of fragments or dust formed as a result of such mechanical attrition.

As measured by the RIT dust test utilizing a rigid, metal material container wall, a mechanically robust granule has an enzyme dust level of less than about 400,000 ng/g, and a reduction in enzyme dust of at least 25%, at least 35%, at least 45%, at least 55%, at least 65%, at least 75%, at least 85%, at least 95%, and at least 99% as compared to a granule without an impregnated plasticizer.

It is a key and surprising feature of this invention that application of a plasticizer at selected temperatures and subsequent to the formation of the particle and polymeric coating, as opposed to concurrent application with the polymer or other ingredients in the granule, results in the plasticizer being absorbed into the interior of the granule and incorporated into the granule without resulting in agglomeration or adhesion of individual particles to each other. Moreover, the polymeric films on such post-plasticized granules are well-formed, flexible, uniform and of high physical integrity. When attempts are made to simultaneously apply polymeric coatings and plasticizers to granules, especially at plasticizer levels above about 5-10% of the polymer, the coatings become wet or tacky, and the particles either agglomerate into multi-particulate aggregates, or tend to adhere and break-up again, resulting in non-uniform coatings with pock marks, chips, or other deformities. Such non-uniformities in the coating layer give rise to elevated dust levels or loss in protection of the active against humidity, oxidizing species, or other environmental stresses. Post-plasticized granules made by the impregnation process of the current invention overcome these deficiencies.

The impregnated plasticizer of the present invention has the advantage of being able to convert otherwise mechanically sensitive granules or cores into mechanically robust particles using plasticizers without unwanted agglomeration or tackiness. It is therefore not necessary to completely re-engineer or reformulate a granule to make it mechanically robust, particularly since the plasticizer is applied to an already formed granule or granule coating. It is a further advantage of this invention that converting mechanically sensitive granules to mechanically robust granules does not diminish desirable properties such as ease of production, handling, solubility, enzymatic stability, thermal stability, and resistance to water pickup during storage in humid conditions.

CORES

The core is the inner nucleus of the granule, and is characterized as a mechanically sensitive particle. Suitable cores for use in the present invention are preferably of a highly hydratable material (i.e., a material which is readily dispersible or soluble in water). The core material should either disperse in water (disintegrate when hydrated) or solublize in water by going into a true aqueous solution. Clays (bentonite, kaolin), nonpareils and agglomerated potato starch are considered dispersible. Nonpareils are spherical particles consisting of a seed crystal that has been built onto and rounded into a spherical shape by binding layers of powder and solute to the seed crystal in a rotating spherical container. Nonpareils are typically made from a combination of a sugar such as sucrose, and a powder such as cornstarch. Alternate seed crystal materials include sodium chloride or sodium sulfate seeds and other inorganic salts which may be built up with ammonium sulfate, sodium sulfate, potassium sulfate and the like.

Particles and granules composed of inorganic salts and/or sugars and/or small organic molecules may be used as the cores of the present invention. Suitable water soluble ingredients for incorporation into cores include: sodium chloride, ammonium sulfate, sodium sulfate, urea, citric acid, sucrose, lactose and the like. Water-soluble ingredients can be combined with water dispersible ingredients. Cores of the present invention may further comprise one or more of the following: active ingredients, polymers, fillers, plasticizers, fibrous materials, extenders and other compounds known to be used in cores. Suitable polymers include - polyvinyl alcohol (PVA), polyethylene glycol, polyethylene oxide, and polyvinyl pyrrolidine. The PVA may be partially hydrolyzed (70 - 90%); intermediately hydrolyzed (90 - 98%); fully hydrolyzed (98 - 99%); super hydrolyzed (99 - 100%) PVA, or a mixture thereof, with a low to high degree of viscosity.

Suitable fillers useful in the cores include inert materials used to add bulk and reduce cost, or used for the purpose of adjusting the intended enzyme activity in the finished granule. Examples of such fillers include, but are not limited to, water soluble agents such as urea, salts, sugars and water dispersible agents such as clays, talc, silicates, carboxymethyl cellulose and starches.

Suitable plasticizers useful in the cores of the present invention are nonvolatile solvents added to a polymer to reduce its glass transition temperature, thereby reducing brittleness and enhancing deformability. Typically, plasticizers are low molecular weight organic compounds and are highly specific to the polymer being plasticized. Examples include, but are not limited to, sugars (such as, glucose, fructose and sucrose), sugar alcohols (such as, sorbitol, xylitol and maltitol) polyols (polyhydric alcohols for example, alcohols with many hydroxyl radical groups such as glycerol, ethylene glycol, propylene glycol or polyethylene glycol), polar low molecular weight organic compounds, such as urea, or other known plasticizers such as dibutyl or dimethyl phthalate, or water.

Suitable fibrous materials useful in the cores of the present invention include materials which have high tensile strength and which can be formed into fine filaments having a diameter of 1 to 50 microns and a length equal to at least four diameters. Typical fibrous materials include, but are not limited to: cellulose, glass fibers, metal fibers, rubber fibers, azlon (manufactured from naturally occurring proteins in com, peanuts and milk) and synthetic polymer fibers. Synthetics include Rayon®, Nylon®, acrylic, polyester, olefin, Saran®, Spandex® and Vinal®. Typically cellulose fibers have an average fiber length of 160 microns with a diameter of about 30 microns.

Cores can be fabricated by a variety of granulation techniques well known in the art including: crystallization, precipitation, pan-coating, fluid-bed coating, rotary atomization, extrusion, spheronization, drum granulation and high-shear agglomeration.

In one embodiment of the present invention, the core is a water-soluble or dispersible nonpareil (either sugar or salt as described above) which can be further coated by or built up from the seed crystal (nonpareil) using polyvinyl-lalcohol (PVA) either alone or in combination with anti-agglomeration agents such as titanium dioxide, talc, or plasticizers such as sucrose or polyols. In general, the core including any active ingredient incorporated therein is a mechanically sensitive particle. However, the invention is not limited by the type of core, and numerous patents and publications describe cores that may be used in the invention and reference is made to US-A-5,879,920; US-A-4,689,287 and WO-A-0024877.

ACTIVE INGREDIENTS

The active ingredient may be any material that is to be added to a granule. The active ingredient may be a biologically viable material, an agrochemical ingredient, such as a pesticide, fertilizer or herbicide; a pharmaceutical ingredient or a cleaning ingredient. In a preferred embodiment, the active ingredient is an enzyme, protein, peptide, bleach, bleach activator, perfume, vitamin, hormone or other biologically active ingredient. Most preferred active ingredients are one or more enzymes. A nonlimiting list of enzymes include proteases, cellulases, lipases, cutinases, oxidases, transferases, reductases, hemicellulases, amylases, esterases, isomerases, pectinases, lactases, peroxidases, laccases and mixtures thereof. Preferred enzymes include those enzymes capable of hydrolyzing substrates (e.g., stains). These enzymes are known as hydrolases, which include, but are not limited to, proteases (bacterial, fungal, acid, neutral or alkaline), amylases (alpha or beta), lipases, cellulases, and mixtures thereof. Particularly preferred enzymes include those sold under the trade names Purafect, Purastar, Properase, Puradax, Clarase, Multifect, Maxacal, Maxapem, and Maxamyl by Genencor International (USP 4,760,025 and WO 91/06637); Alcalase, Savinase, Primase, Durazyme, Duramyl, and Termamyl sold by Novo Industries A/S (Denmark). Particularly preferred proteases are subtilisins. Cellulase is another preferred enzyme and particularly cellulases or cellulase components isolated from *Trichoderma reesei*, such as found in the product Clazinase. Preferred amylases include alpha amylases obtained from *Bacillus licheniformis.*

In one aspect, one or more active ingredients are incorporated in the core of the granule, in another preferred aspect one or more active ingredients are layered around the core, and in another aspect the active ingredients are in the coating. When layered around the core, the layer comprising the active ingredient may additionally include a binder such as a polymer as mentioned herein, preferably a vinyl polymer such as PVA.

The layer comprising the active ingredient may further comprise other plasticizers and anti-agglomeration agents added concurrently with other granule ingredients. Suitable nonlimiting examples of plasticizers useful in the present invention include polyols such as sugars, sugar alcohols or polyethylene glycols (PEGs) having a molecular weight less than 1000, ureas or other known plasticizers, such as dibutyl or dimethyl phthalate, or water. Suitable anti-agglomeration agents include fine insoluble material such as talc, $TiO_2$, clays and amorphous silica.

The granules of the invention may include between 0.01 to 50% by weight active ingredient. Particularly preferred are enzymes comprising at least 0.5%, at least 5%, at least 10%, at least 20%, at least 30% and up to and including 40%. The layer comprising the active ingredient, including any nonenzyme solids and binders therein, may comprise between about 0.01 to 50%, about 0.05 to 35%, about 0.1 to 15% and about 0.5 to 8.0% by weight of the granule.

COATINGS

While one skilled in the art is aware of water-soluble polymers and water dispersible polymers, in general a water-soluble polymer will have a solubility of at least 1%, preferably at least 5%, and frequently at least 15% in deionized water at room temperature. Water dispersible polymers are those which break up into fine particles of no greater than about 50 microns at room temperature within about 10 minutes of moderate agitation in deionized water or a solution of less than about 5% of a detergent or nonionic surfactant. Moderate agitation may be achieved for example by use of a stir bar at 200 rpm in a 200 ml beaker filled to 100 ml with aqueous solvent.

Preferred nonlimiting polymers are water-soluble and water dispersible polymers that may be plasticized according to the invention and are selected from polyvinyl alcohols (PVA), modified PVA as described, for example, in U.S. Patent App. Ser. No. 09/12/2001, published on 08/01/2002, polyethylene glycols (PEG), polyethylene oxides (PEO), polyvinyl pyrrolidones (PVP), cellulose ethers, alginates, gelatin, modified starches and substituted derivatives, hydrolysates and copolymers thereof. Most preferred polymers are PVA, cellulose ethers, such as methyl cellulose and hydroxylpropyl cellulose, gelatin and modified starches, such as hyproxypropyl starch produced from cornstarch. Mostly preferred is PVA, however, it is not intended that the present invention be limited to any particular polymer. The polymers may be utilized in a foamed morphology. If PVA is used, in preferred embodiment the polymer has a level of hydrolysis in the range of about 50 to 99%, at least about 80%, at least about 85%, at least about 90%, and at least about 95%. The polymer may have an average molecular weight of about 4,000 to 250,000, preferably from 5,000 to 200,000; also from 10,000 to 100,000. For the purpose of the invention, a polymer comprising the flexible film may have a suitable viscosity below about 2000 cps, below 1000 cps and even below 500 cps at a temperature range of about 25 to 90°C. Suitable polymers also include natural and synthetic gelling agents. Nonlimiting examples include hydrocolloids or gums, such as gelatin, pectin, carrageenan, xanthan gum, gum arabic, alginate, agarose, or any combination thereof. These gelling agents may also be combined with the polymers as listed above. The polymer generally constitutes about 0.5% to about 50% by weight of the granule, and about 1% to 100% of the film forming coating prior to addition of the plasticizer.

In other embodiments, the coating may be or include surfactants, powders, clays, talc, titanium dioxide, and fibers.

Further, cross linking agents may be added to gel or modify the properties of the coating or film and reduce or delay its solubility, for example boric acid may be used to cross link PVA and calcium salts may be used to cross link sodium alginate.

PLASTICIZERS

Suitable plasticizers which are impregnated into the granule are nonvolatile solvents which reduce the Tg and brittleness and enhance deformability of the granule. The plasticizers are low molecular weight organic compounds with molecular weights below 1000, and are selected from polyhydric alcohols, for example alcohols with many hydroxyl groups such as glycerol, glycerin, ethylene glycol, diethylene glycol, triethylene glycol, propylene glycol, dipropylene glycol, polyethylene glycol, polar low molecular weight organic compounds, such as urea, sugars, sugar alcohols, oxa diacids, diglycolic acids linear carboxylic acids with at least one ether group, dibutyl or dimethyl phthalate. Sugars may include but are not limited to sucrose, dextrose, fructose, maltose, trehalose, and raffinose. Sugar alcohols that may serve as plasticizers and include sorbitol, xylitol, and maltitol. Other plasticizers are ethanolacetamide, ethanolformamide, triethanolamine acetate, sodium thiocyanates, and ammonium thiocyanates. Most preferred are glycerol, triethylene glycol, propylene glycol, sorbitol, and polyethylene glycol having an average molecular weight below 600. The plasticizer is preferably present at a level of 0.05 to 25% by weight of the granule, preferably 1 to 10% by weight of the granule; and more preferably 0.1 to 5.0% by weight of the granule. The exact level will depend on the polymeric material comprising the film. Generally, the ratio of plasticizer to polymer ranges from 0.05 to 5.0.

The impregnated mechanically robust granule may also include further components such as, but not limited to fillers, lubricants, and pigments. These compounds are well known to one of ordinary skill in the art and are further discussed herein.

In one embodiment of the invention a mechanically sensitive particle is converted to an impregnated mechanically robust granule. This is achieved by applying the plasticizer to an outer coating of a mechanically sensitive particle. One skilled in the art can determine a mechanically sensitive particle by standard tests known in the art and as described and defined herein.

One specific non-limiting example includes the T-granulation process of Novo-Nordisk which provides for the inclusion within a composition undergoing granulation, of finely divided cellulose fibers, salts and binders added to enzymes and formed into granules using high shear granulators or drum granulators. In addition a waxy substance can be used to coat the granules and further coating layers may be applied (See US-A-4,106,991). Even though the obtained granule is tough and somewhat resistant to compression, it is not very resistant to repeated impact forces (See US-A-5, 324,649) and is considered a mechanically sensitive particle according to the definition herein. An impregnated film according to the invention applied to the T-granule may convert the T-granule from a mechanically sensitive particle to an impregnated mechanically robust granule according to the present invention.

OTHER LAYERS

The granules of the present invention which include the impregnated plasticizer may further comprise one or more other coating layers. For example, such coating layers may be one or more intermediate coating layers applied prior to application of the plasticizer. Additionally, one or more coating layers may be one or more over-coating layers, wherein a coating is applied prior to application of the plasticizer. A combination of one or more intermediate coating layers and one or more over-coating layers may also comprise the granules. Coating layers may serve any of a number of functions depending on the end use of the granule. For example, coatings may render the active ingredient, particularly enzymes, resistant to oxidation by bleach, or coating layers may bring about the desirable rate of dissolution upon introduction of the granule into an aqueous medium, or provide a further barrier against ambient moisture in order to enhance the storage stability of the granule and reduce the possibility of microbial growth within the granule.

In an embodiment of the present invention, the coating layer comprises one or more polymer(s) and, optionally, a low residue pigment or other excipients such as lubricants. Such excipients are known to those skilled in the art.

Furthermore, coating agents may be used in conjunction with other active agents of the same or different categories.

Suitable polymers include PVA and/or PVP or mixtures of both. If PVA is used, it may be partially hydrolyzed, fully hydrolyzed or intermediately hydrolyzed PVA having low to high degrees of viscosity (preferably partially hydrolyzed PVA having low viscosity). Other vinyl polymers which may be useful include polyvinyl acetate and polyvinyl pyrrolidone. Useful copolymers include, for example, PVA-methylmethacrylate copolymer. Other polymers such as PEG may also be used in the outer layer. These further coating layers may further comprise one or more of the following: plasticizers, pigments, lubricants such as surfactants or antistatic agents and, optionally, additional enzymes. Suitable plasticizers useful in the coating layers of the present invention are those disclosed herein above. Suitable pigments useful in the coating layers of the present invention include, but are not limited to, finely divided whiteners such as titanium dioxide or calcium carbonate, or colored pigments, or a combination thereof. Preferably such pigments are low residue pigments upon dissolution.

As used herein "lubricants" mean any agent which reduces surface friction, lubricates the surface of the granule, decreases static electricity or reduces friability of the granules. Lubricants can also play a related role in improving the coating process, by reducing the tackiness of binders in the coating. Thus, lubricants can serve as anti-agglomeration agents and wetting agents.

Suitable lubricating agents include, but are not limited to, surfactants (ionic, nonionic or anionic), fatty acids, antistatic agents and antidust agents. Preferably the lubricant is a surfactant, and most preferably is an alcohol-based surfactant such as a linear, primary alcohol of a 9 to 15 carbon atom chain length alkane or alkene or an ethoxylate or ethoxysulfate derivative thereof. Such surfactants are commercially available as the Neodol® product line from Shell International Petroleum Company. Other suitable lubricants include, but are not limited to, antistatic agents such as StaticGuard™, Downey™, Triton X100 or 120 and the like, antidust agents such as Teflon™, or other lubricants known to those skilled in the art.

Other intermediate layers, such as binders, structuring agents, and barrier layers may be included. Suitable barrier materials include, for example, inorganic salts, sugars, or organic acids or salts. Structuring agents can be polysaccharides or polypeptides. Preferred structuring agents include starch, modified starch, carrageenan, cellulose, modified cellulose, gum arabic, guar gum, acacia gum, xanthan gum, locust bean gum, chitosan, gelatin, collagen, casein, polyaspartic acid and polyglutamic acid. Preferably, the structuring agent has low allergenicity. A combination of two or more structuring agents can be used in the granules of the present invention. Binders include but are not limited to sugars and sugar alcohols. Suitable sugars include but are not limited to sucrose, glucose, fructose, raffinose, trehalose, lactose and maltose. Suitable sugar alcohols include sorbitol, mannitol and inositol.

OTHER ADJUNCT INGREDIENTS

Adjunct ingredients may be added to the granules of the present invention, including but not limited to: metallic salts, solubilizers, activators, antioxidants, dyes, inhibitors, binders, fragrances, enzyme protecting agents/scavengers such as ammonium sulfate, ammonium citrate, urea, guanidine hydrochloride, guanidine carbonate, guanidine sulfonate, thiourea dioxide, monethyanolamine, diethanolamine, triethanolamine, amino acids such as glycine, sodium glutamate and the like, proteins such as bovine serum albumin, casein and the like, etc., surfactants, including anionic surfactants, ampholytic surfactants, nonionic surfactants, cationic surfactants and long-chain fatty acid salts, builders, alkalis or inorganic electrolytes, bleaching agents, bluing agents and fluorescent dyes, and caking inhibitors. These surfactants are described in PCT Application PCT/US92/00384.

PROCESSES FOR MAKING THE GRANULE WITH AN IMPREGNATED PLASTICIZER

In general, methods well known in the art of enzyme granulation, including fluidized bed-spray-coating, pan-coating and other techniques may be used for making part of the granule according to the invention, including the core, active ingredient layer and optionally intermediate or over-coating layers. However, surprisingly it has been found that the means of applying the plasticizer may be a critical step in providing a granule according to the invention herein having improved characteristics such as mechanical robustness, low dust and increased stability.

A preferred process for applying the plasticizer film herein comprises applying the plasticizer in liquid form onto the formed granule, or to a formed coating on the granule, at 65°C to 85° C, which is well above the glass transition temperature of the polymer. The plasticizer is not applied as a mixture with other granule ingredients.

The completed granule should remain stable and continuous and not be so soft or tacky so as to render the granule unhandleable. A stable granule is one wherein the plasticizer is impregnated into the granule and the granule is free flowing, easy to handle and not tacky.

In one embodiment, one or more active ingredients will be incorporated into the core and in another embodiment one or more active ingredients will comprise a layer surrounding the core.

COMPOSITIONS COMPRISING THE HIGHLY MECHANICALLY ROBUST GRANULE.

The granules according to the invention may be incorporated in any number of compositions which require active ingredients to be protected against inactivation by elevated temperature, humidity or exposure to denaturants, oxidants or other harsh chemical and physical forces. In particular, the granules are useful in cleaning compositions, fabric care compositions, personal care compositions and pharmaceutical compositions. Preferred compositions include detergent compositions including laundry and dishwashing compositions. The compositions typically include one or more compounds particularly surfactants (See WO 9206165). Pharmaceutical compositions and personal care compositions including one or more additives are also preferred.

EXPERIMENTAL

EXAMPLE 1 - IMPREGNATED MECHANICALLY ROBUST PARTICLES INCLUDING A CORE AND ACTIVE INGREDIENT.

Core particles were prepared by charging sodium sulfate seeds or cores into a Glatt 3 fluidized bed coater, and spraying the cores with ammonium sulfate followed by a coating of polyvinyl alcohol and talc. The cores contained 48% of a 31.5% sodium sulfate seed, a 62.5% ammonium sulfate layer, and a coating of 2% PVA (Moviol 3-83, Clariant, Charlotte, NC) and 4% talc. A solution of ultrafiltration concentrate of subtilisin protease containing 28.2% protease solids plus 1% PVA (Elvanol 90-50) was sprayed onto the coated cores. Then a solution of 10% ammonium sulfate was added. The coating layer, 13% by weight of the total granule, was 5.2% PVA (Elvanol 90-50, from Dow), 6.5% $TiO_2$ and 1.3% Neodol 23-6.5T Shell Chemical nonionic surfactant.

Onto the granules, encompassing the already formed coating layer described above, an aqueous solution of 80% w/w glycerol (8 parts of glycerol to 2 parts of water) was sprayed, repeated in several different runs at bed temperatures varying between 65° C to 85° C. Sufficient glycerol solution was applied until the glycerol constituted 10% by weight of the granule. Finally, 1 % talc was dusted onto selected samples for the purpose of improving flowability of the granules. The glycerol solution was applied under the following conditions: fluidization airflow: 100 cfm; atomization air pressure: 275790 Pa (40 psi); bed temperature: between 65° C to 85° C; inlet air temperature: 10-12 degrees above the bed temperature; spray rate: 5 grams/minute; total application time: 75 minutes.

Six samples of the coated granules were prepared having enzyme content varying between about 69-80 mg/kg. Granule 1 was a control granule without the plasticized glycerol coating, and the glycerol was added to granules 2-6 at temperatures varying between 65 - 85° C. The ratio of glycerol to the polymer in the coating was about 1.9.

The granules were tested for impact strength in the RIT dust test. About 30 mg of granules were placed into a rigid, plastic material box of dimensions 2 cm x 3 cm x 1.5 cm and oscillated up and down at a frequency of 60 Hz causing the granules to impact the walls of the box at an impact velocity of 8.52 meters/second. The box was sealed to completely contain all of the dust generated during the test procedure. The test was run during 30 minutes resulting in 216,000 impacts or collisions with the box walls. At various time intervals (60, 120, 240, 420, 600, 900, 1200 and 1800 seconds, the box was opened and the content of the box was sieved through a 300 $\mu$m sieve to remove any fines or damaged particles. The results of RIT dust are reported in Table 1. The impregnated glycerol plasticizer drastically reduced RIT dust by at least 99% as compared to the control without the glycerol. The percent reduction was calculated according to the following formula:

$$100 - 100X(1\text{-final wt/starting wt}).$$

Table 1

| Sample | Enzyme mg/kg | Glycerol Amount | Temperature | Talc | RIT Enzyme Dust ng/gram | % Enzyme Dust Reduction |
|---|---|---|---|---|---|---|
| 1 | 80.0 | None | | yes | 3,110,577 | |
| 2 | 72.7 | 10% | 65 | yes | 12,690 | 99.6 |
| 3 | 72.2 | 10% | 70 | yes | 4,096 | 99.9 |
| 4 | 71.7 | 10% | 70 | no | 3,182 | 99.9 |
| 5 | 69.3 | 10% | 75 | yes | 9,313 | 99.7 |

(continued)

| Sample | Enzyme mg/kg | Glycerol Amount | Temperature | Talc | RIT Enzyme Dust ng/gram | % Enzyme Dust Reduction |
|--------|--------------|-----------------|-------------|------|-------------------------|-------------------------|
| 6 | 74.0 | 10% | 85 | yes | 22,270 | 99.3 |

EXAMPLE 2 - HEUBACH AND ELUTRIATION DUST TESTING OF ANOTHER IMPREGNATED COATING APPLIED TO AN IMPACT SENSITIVE PARTICLE CONTAINING AN ACTIVE INGREDIENT

Into a Glatt 30 fluid bed coater were charged 10.0 kg of Purafect 3450M™ protease enzyme granules from Genencor International, Inc., Palo Alto, California. The Purafect enzyme granules were comprised of 25% sucrose crystal cores; 28.2% of a mixture of protease solids from ultrafiltrate, corn starch and sucrose; a 20% magnesium sulfate heptahydrate layer, and a 12.3 wt.% outer coating layer of 2.5% Methocel A-15 (Dow), 2.5% Pure Cote B 790 (Grain Processing), 1.3% Neodol 23-6.5T (Shell), and 6% titanium dioxide.

Samples of the granules were then coated with glycerol (15% by weight of the granule) under the following conditions: fluidization airflow: 2.8 m3/min; atomization air pressure: 344740 Pa (50 psi) atomization air temperature: 127°C; bed temperature: 90° C; spray rate: 7 grams/minute; and total application time: 205 minutes. The ratio of glycerol to polymer was about 3.0. The starting granules and the granules coated with glycerol were measured for enzyme dust using the Heubach and Elutriation dust tests. The results are presented in Table 2. The results show that the granule with the glycerol plasticizer had about 86% reduction in enzyme dust as compared to the control granule in the Elutriation test.

Table 2

| Sample | Enzyme Payload GSU/g | Glycerol Added | Temperature | Talc | Heubach Total Dust mg/pad | Heubach Enzyme Dust GSU/pad | Elutriation Dust mGSU/pad | % Dust Reduction |
|--------|----------------------|----------------|-------------|------|----------------------------|------------------------------|----------------------------|------------------|
| 7 | 41.1 | No | | no | | | 2.5 | |
| 8 | 41.4 | 15% | 90 | no | 0.35 (.026 mg/gram) | <.0004 | 0.35 | 86 |

EXAMPLE 3 - HEUBACH DUST TESTING OF A GLYCEROL IMPREGNATED MECHANICALLY ROBUST COATING APPLIED TO A PARTICLE CONTAINING AN ACTIVE INGREDIENT

Into a Glatt FL-1 Fluid Bed Coater were charged 2.2 Kg of granules containing the enzyme protease from Genencor International, Inc., Palo Alto, California. The enzyme granules were comprised of 25% sodium sulfate cores (50- 70 mesh); 5% of a mixture of protease solids, talc and Mazu DF6000K (Dow-Corning); a 63.6% sodium sulfate layer, and a 6.4% by weight of the granule outer coating layer of 2.4% PVA (Elvanol 51-05, Dupont, Wilmington, DE), 1% Neodol 23-6.5T (Shell), and 3% titanium dioxide.

Onto granules comprising the above formed polymeric, a 10% glycerol solution (1 parts of glycerol to 9 parts of water) was sprayed, repeated in separate runs at bed temperatures varying between 45°C to 50°C. These granules were coated with glycerol under the following conditions: fluidization airflow: 85 cfm; atomization air pressure: 275790 Pa (40 psi); bed temperature: between 45°C to 50°C; inlet air temperature: between 70° C to 80° C; spray rate: 10 grams/ minute; total application time: 75 minutes.

Sample granules were impregnated with glycerol, applied to the starting granules in separate runs at varying glycerol levels, as indicated in Table 3, and these granules were measured for enzyme dust in the Heubach dust test. The ratio of glycerol to polymer, varied from about 0.14 to about 1.2 in the different runs. The results are shown in Table 3 which shows that Heubach enzyme dust and Heubach total dust are reduced more than two orders of magnitude with the impregnation of 2.85% glycerol.

Table 3

| Sample No. | %Glycerol | Heubach Total Dust mg/gram | Heubach Enzyme Dust ug/gram | % Enzyme Dust Reduction |
|------------|-----------|----------------------------|------------------------------|-------------------------|
| 9 | 0 | 0.100 | 4.90 | |

(continued)

| Sample No. | %Glycerol | Heubach Total Dust mg/gram | Heubach Enzyme Dust ug/gram | % Enzyme Dust Reduction |
|---|---|---|---|---|
| 10 | 0.34 | 0.051 | 1.96 | 60 |
| 11 | 0.68 | 0.029 | 0.41 | 91.6 |
| 12 | 1.36 | 0.007 | 0.02 | 99.6 |
| 13 | 2.27 | 0.007 | 0.02 | 99.6 |
| 14 | 2.85 | 0.000 | 0.18 | 96.3 |

EXAMPLE 4 - HEUBACH DUST TESTING OF A TRIETHYELENE GLYCOL IMPREGNATED MECHANICALLY ROBUST COATING APPLIED TO A PARTICLE CONTAINING AN ACTIVE INGREDIENT WITH A THIN COATING

Into a Glatt FL-1 Fluid Bed Coater were charged 2.2 Kg of protease containing granules from Genencor International, Inc., Palo Alto, California. The enzyme granules were comprised of 25% sodium sulfate cores (50- 70 mesh); 5% of a mixture of protease solids , Talc and Mazu DF6000K (Dow-Corning); a 54.1 % sodium sulfate layer, and an outer coating layer of 2.4% PVA (Elvanol 51-05, Dupont, Wilmington, DE), 1% Neodol 23-6.5T (Shell), and 3% titanium dioxide.

Onto samples of the coated granules, a 10% triethylene glycol and 5% TiO2 solution (150 grams of glycerol and 75 grams TiO2 in 1500 mls of water) was sprayed at bed temperatures varying between 45°C to 50°C. These granules were coated with triethylene glycol under the following conditions: fluidization airflow: 85 cfm; atomization air pressure: 275790 Pa (40 psi); bed temperature: between 45°C to 50°C; inlet air temperature: between 70° C to 80° C; spray rate: 10 grams/minute; total application time: 75 minutes.

Granules were impregnated with a range of triethylene glycol levels and these granules were measured for enzyme dust with the Heubach test. The ratio of triethylene glycol to polymer ranged from about 0.31 to about 2.8. These results are shown in Table 4 which shows that Heubach enzyme dust is reduced more than two orders of magnitude with the impregnation of 3.40% triethylene glycol while the total Heubach dust is reduced more than 5 times.

Table 4

| Sample Number | %Triethylene Glycol | Heubach Total Dust mg/gram | Heubach Enzyme Dust ug/gram | % Enzyme Dust Reduction |
|---|---|---|---|---|
| 20 | 0 | 0.126 | 5.46 | |
| 21 | 0.75 | 0.067 | 2.55 | 53.3 |
| 22 | 1.36 | 0.030 | 0.27 | 95.1 |
| 23 | 3.4 | 0.022 | 0.05 | 99.1 |
| 24 | 6.8 | 0.022 | 0.04 | 99.3 |

EXAMPLE 5 - RIT AND HEUBACH DUST TESTING OF A GLYCEROL IMPREGNATED MECHANICALLY ROBUST COATING APPLIED TO A PARTICLE CONTAINING A METHYLCELLULOSE COATING AND AN ACTIVE INGREDIENT

Into a Fluid Bed Coater were charged 100 Kg of granules containing protease from Genencor International, Inc., Palo Alto, California. The enzyme granules were comprised of 69% salt cores; 15% protease solids and 0.5% PVA; and a 13% coating of methylcellulose, TiO2 and Neodol (0.7%).

Onto the formed coat of the granules, a glycerol solution between 5 and 50% was sprayed at bed temperatures of about 70° C. These granules were coated with glycerol under the following conditions: fluidization airflow: between 32000 and 33000 Nm$^3$ /hr; atomization air pressure: 275790 Pa (40 psi); bed temperature: 7 between 45 and 55° C; spray rate: between 509 l/hour and 1018 l/hour; total application time: 15 minutes.

Granules were impregnated with a range of glycerol levels and these granules were measured for enzyme dust with the Heubach test and the RIT test. The ratio of glycerol to polymer was about 0.07 to 0.8. The RIT test was performed as described above, except that the 30 mgs of granules were vibrated for 30 minutes at a frequency of 30 Hz and an impact velocity of 3.2 meters/second, resulting in 108,000 collisions. These results are shown in Table 5 which shows that RIT enzyme dust was reduced about 88 to 95 percent with the addition of 4% glycerol, and Heubach enzyme dust is reduced with the addition of 4% glycerol while the total Heubach dust is reduced by a factor of 4 times under the same conditions. The Heubach enzyme dust values for Sample No. 25 did not indicate that the granule was impact sensitive, however, the Sample 25 values measured by the RIT test do show that this sample was impact sensitive.

Table 5

| Sample Number | %Glycerol | Heubach Total Dust mg/gram | Heubach Enzyme Dust ug/gram | % Enzyme Dust Reduction | RIT Dust Enzyme ng/gram |
|---|---|---|---|---|---|
| 25 | 0 | 0.03 | 1.1 | | 1,924,643 |
| 26 | 0.34 | 0.007 | 1.1 | | |
| 27 | 0.68 | 0.015 | 0.47 | | |
| 28 | 1.36 | 0.0074 | 0.09 | | |
| 29 | 2.0 | 0.022 | 0.13 | | |
| 30 | 2.7 | 0.007 | 0.13 | | |
| 31 | 4.0 | 0.000 | 0.05 | 95 | 104,858 |
| 32 | 4.0 | | | 88.4 | 223,245 |

EXAMPLE 6 - RIT DUST TESTING OF A GLYCEROL IMPREGNATED MECHANICALLY ROBUST COATING APPLIED TO A PARTICLE CONTAINING A METHYLCELLULOSE COATING AND AN ACTIVE INGREDIENT.

Additional samples similar to those of Example 5 were tested in a Fluid Bed Coater charged with 100 Kg of commercial granules containing the enzyme protease from Genencor International, Inc., Palo Alto, California. The enzyme granules were comprised of 75.5% non-pareil cores; 10/7% protease solids; a 13% coating of methylcellulose (4.6%), $TiO_2$ (5.8%), PEG 600 (1.6%) and Neodol 23-6.5-T (0.9%), with an additional 0.8% overcoat of Neodol 23-6.5-T.

Onto the formed coat of the granules, a glycerol solution between 5 and 50% was sprayed at bed temperatures of about 70° C. These granules were coated with glycerol under the following conditions: fluidization airflow: between 32000 and 33000 $Nm^3$ /hr; atomization air pressure: 275790 Pa (40 psi); bed temperature: 7 between 45 and 55° C; spray rate: between 509 l/hour and 1018 l/hour; total application time: 15 minutes.

Granules were impregnated with 4% glycerol and these granules were measured for enzyme dust with the RIT test. The ratio of glycerol to polymer was 0.87. The RIT test was performed as described above, except that the 30 mgs of granules were vibrated for 30 minutes at a frequency of 30 Hz in a plastic material rigid container, at an impact velocity of 3.2 meters/second, resulting in 108,000 collisions. These results are shown in Table 6 which shows that RIT enzyme dust is reduced by 12 times with the addition of 4% glycerol.

Table 6

| Sample Number | % Glycerol | % Enzyme Dust Reduction | RIT Dust Enzyme ng/gram |
|---|---|---|---|
| 33 | 0 | 0 | 1,924,643 |
| 34 | 4.0 | 91.5 | 164,051 |

EXAMPLE 7. T GRANULE CONVERTED BY THE ADDITION OF GLYCEROL

Commercially available T Granules sold as Savinase 12 TXT (Novozymes) were obtained and fluidized for the addition of varying amounts of glycerol. The RIT test was performed with 30 mgs of granules vibrated for 30 minutes at a frequency of 30 Hz in a plastic material rigid container, at an impact velocity of 3.2 meters/second, resulting in 108,000 collisions. The RIT results set out below in Table 6 demonstrate that adding an impregnated coating reduces RIT dust.

TABLE 7

| Granule | % Glycerol | RIT Dust ng/gram | RIT Dust % Reduction |
|---|---|---|---|
| Savinase 12TXT | 0 | 2,127,840 | |

(continued)

| Granule | % Glycerol | RIT Dust ng/gram | RIT Dust % Reduction |
|---------|-----------|------------------|----------------------|
| Savinase 12TXT | 2.5 | 1,261,260 | 43.2 |
| Savinase 12TXT | 2.5 | 1,827,540 | 42.3 |

## Claims

1. A method of making a mechanically robust granule comprising:

   a) providing a formed granule having an active ingredient and a water-soluble or water dispersible polymer as a formed film surrounding the active ingredient; and
   b) applying a plasticizer to the formed granule at a temperature above the glass transition temperature of the polymer until at least 50% of the plasticizer is absorbed into the granule, wherein the plasticizer has a molecular weight of less than 1000 and is selected from polyhydric alcohols, urea, sugars, sugar alcohols, oxa diacids, diglycolic acids, linear carboxylic acids with at least one ether group, dibutyl or dimethyl phthalate, ethanolacetamide, ethanolformamide, triethanolamine acetate, sodium thiocyanates, and ammonium thiocyanates.

2. The method of claim 1, wherein the plasticizer is glycerol, triethylene glycol, propylene glycol, sorbitol, and polyethylene glycol.

3. The method of claim 1, wherein the plasticizer applied in step b) comprises 0.05 to 25% w/w of the granule.

4. The method of claim 1, wherein the active ingredient is an enzyme.

5. The method of claim 1, wherein the polymer is selected from polyvinyl alcohols, polyethylene glycols, polyethylene oxides, polyvinyl pyrrolidones, cellulose ethers, alginates, gelatin, modified starches and substituted derivatives, hydrolysates and copolymers thereof.

6. The method of claim 1, wherein step a) comprises selecting a water soluble or dispersible core material which comprises an active ingredient, coating the core material with the polymer, and allowing the polymer to form a film prior to performing step b).

7. The method of claim 1, wherein step a) comprises selecting a water soluble or dispersible core material, surrounding the core material with the active ingredient, coating the polymer over the active ingredient, and allowing the polymer to form a film prior to performing step b).

8. The method of claim 1, wherein step b) comprises applying the plasticizer until at least 99 % of the plasticizer is absorbed into the granule.

9. The method of claim 1, wherein step b) is performed at 65 °C to 90°C.

10. The method of claim 1, wherein step b) is performed at 45°C to 90°C.

11. The method of claim 1, wherein step b) is performed at 65°C to 85°C.

12. A mechanically robust granule obtainable by the method of claim 1.

13. A cleaning composition comprising the granule of claim 12.

14. A personal care composition comprising the granule of claim 12.

## Patentansprüche

1. Verfahren zur Herstellung einer mechanisch robusten Granalie, umfassend:

a) Bereitstellen einer gebildeten Granalie, die einen Wirkstoff und ein wasserlösliches oder wasserdispergierbares Polymer als einen gebildeten, den Wirkstoff umgebenden Film umfasst; und

b) Aufbringen eines Weichmachers auf die gebildete Granalie bei einer Temperatur oberhalb der Glasübergangstemperatur des Polymers, bis wenigstens 50% des Weichmachers in der Granalie absorbiert sind, wobei der Weichmacher ein Molekulargewicht von weniger als 1000 aufweist und ausgewählt ist aus Polyalkoholen, Harnstoff, Zuckern, Zukkeralkoholen, Oxadisäuren, Diglycolsäuren, linearen Carbonsäuren mit wenigstens einer Ethergruppe, Dibutyl- oder Dimethylphthalat, Ethanolacetamid, Ethanolformamid, Triethanolaminacetat, Natriumthiocyanaten und Ammoniumthiocyanaten.

2. Verfahren nach Anspruch 1, wobei der Weichmacher Glycerol, Triethylenglycol, Propylenglycol, Sorbitol und Polyethylenglycol ist.

3. Verfahren nach Anspruch 1, wobei der in Schritt b) aufgebrachte Weichmacher 0,05 bis 25% (w/w) der Granalie umfasst.

4. Verfahren nach Anspruch 1, wobei der Wirkstoff ein Enzym ist.

5. Verfahren nach Anspruch 1, wobei das Polymer ausgewählt ist aus Polyvinylalkoholen, Polyethylenglycolen, Polyethylenoxiden, Polyvinylpyrrolidonen, Celluloseethern, Alginaten, Gelatine, modifizierten Stärken und substituierten Derivaten, Hydrolysaten und Copolymeren davon.

6. Verfahren nach Anspruch 1, wobei der Schritt a) das Auswählen eines wasserlöslichen oder wasserdispergierbaren Kernmaterials, das einen Wirkstoff umfasst, das Beschichten des Kernmaterials mit dem Polymer, und das Ausbildenlassen des Polymers in einen Film vor dem Ausführen des Schritts b) umfasst.

7. Verfahren nach Anspruch 1, wobei der Schritt a) das Auswählen eines wasserlöslichen oder wasserdispergierbaren Kernmaterials, das Umgeben des Kernmaterials mit dem Wirkstoff, das Beschichten des Polymers über den Wirkstoff, und das Ausbildenlassen des Polymers in einen Film vor dem Ausführen des Schritts b) umfasst.

8. Verfahren nach Anspruch 1, wobei der Schritt b) das Aufbringen des Weichmachers, bis wenigstens 99% des Weichmachers in der Granalie absorbiert sind, umfasst.

9. Verfahren nach Anspruch 1, wobei der Schritt b) bei 65°C bis 90°C durchgeführt wird.

10. Verfahren nach Anspruch 1, wobei der Schritt b) bei 45°C bis 90°C durchgeführt wird.

11. Verfahren nach Anspruch 1, wobei der Schritt b) bei 65°C bis 85°C durchgeführt wird.

12. Mechanisch robuste Granalie, erhältlich durch das Verfahren nach Anspruch 1.

13. Reinigungszusammensetzung, umfassend die Granalie nach Anspruch 12.

14. Körperpflegezusammensetzung, umfassend die Granalie nach Anspruch 12.

**Revendications**

1. Procédé de fabrication d'un granulé robuste sur le plan mécanique comprenant :

a) la fourniture d'un granulé moulé ayant un ingrédient actif et un polymère soluble dans l'eau ou miscible dans l'eau sous la forme d'un film moulé entourant l'ingrédient actif ; et

b) l'application d'un plastifiant sur le granulé moulé à une température supérieure à la température de transition vitreuse du polymère jusqu'à ce que au moins 50 % du plastifiant soit absorbé dans le granulé, dans lequel le plastifiant a un poids moléculaire inférieur à 1 000 et est choisi parmi les polyols, l'urée, les sucres, les alcools glucidiques, les diacides oxa, les acides diglycoliques, les acides carboxyliques linéaires avec au moins un groupe éther, le dibutyl- ou le diméthylphtalate, l'éthanolacétamide, l'éthanolformamide, l'acétate de triéthanolamine, les thiocyanates de sodium et les thiocyanates d'ammonium.

**2.** Procédé selon la revendication 1, dans lequel le plastifiant est le glycérol, le triéthylèneglycol, le propylèneglycol, le sorbitol, et le polyéthylèneglycol.

**3.** Procédé selon la revendication 1, dans lequel le plastifiant appliqué au cours de l'étape b) comprend de 0,05 à 25 % en masse pour masse du granulé.

**4.** Procédé selon la revendication 1, dans lequel l'ingrédient actif est une enzyme.

**5.** Procédé selon la revendication 1, dans lequel le polymère est choisi parmi les alcools polyvinyliques, les polyéthylèneglycols, les oxydes de polyéthylène, les polyvinylpyrrolidones, les éthers de cellulose, les alginates, la gélatine, les amidons modifiés et des dérivés substitués, les hydrolysates et les copolymères de ceux-ci.

**6.** Procédé selon la revendication 1, dans lequel l'étape a) comprend la sélection d'un matériau de noyau soluble ou miscible dans l'eau qui comprend un ingrédient actif, le revêtement du matériau du noyau avec le polymère et le fait de laisser le polymère former un film avant d'effectuer l'étape b).

**7.** Procédé selon la revendication 1, dans lequel l'étape a) comprend la sélection d'un matériau de noyau soluble ou miscible dans l'eau, l'entourage du matériau du noyau avec l'ingrédient actif, le revêtement du polymère sur l'ingrédient actif et le fait de laisser le polymère former un film avant d'effectuer l'étape b).

**8.** Procédé selon la revendication 1, dans lequel l'étape b) comprend l'application du plastifiant jusqu'à ce que au moins 99 % du plastifiant soit absorbé dans le granulé.

**9.** Procédé selon la revendication 1, dans lequel l'étape b) est effectuée entre 65°C et 90°C.

**10.** Procédé selon la revendication 1, dans lequel l'étape b) est effectuée entre 45°C et 90°C.

**11.** Procédé selon la revendication 1, dans lequel l'étape b) est effectuée entre 65°C et 85°C.

**12.** Granulé robuste sur le plan mécanique pouvant être obtenu grâce au procédé selon la revendication 1.

**13.** Composition de nettoyage comprenant le granulé de la revendication 12.

**14.** Composition de soin corporel comprenant le granulé de la revendication 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4106991 A
- US 4242219 A
- US 4689297 A
- US 5324649 A
- US 5879920 A
- US 20020119201 A1
- US 6035716 A
- WO 9803849 A
- US 4689287 A
- WO 0024877 A
- US 4760025 A
- WO 9106637 A
- US 09122001 A
- US 9200384 W
- WO 9206165 A

### Non-patent literature cited in the description

- Enzymes In Detergency. Marcel Dekker, Inc, 1997, 310-312